# EUROPÄISCHE PATENTSCHRIFT

(11) **EP 0 689 441 B1**
(45) Veröffentlichungstag und Bekanntmachung des Hinweises auf die Patenterteilung: **30.05.2001**
(21) Anmeldenummer: 94911065.4
(22) Anmeldetag: 19.03.1994
(51) Int. Cl.: A61K 31/557, A61K 9/107

(54) **PHARMAZEUTISCHE ZUSAMMENSETZUNG IN FORM EINES KITS ENTHALTEND PROSTAGLANDIN E1**
PHARMACEUTICAL COMPOSITION IN FORM OF A KIT COMPRISING PROSTAGLANDIN E1
COMPOSITION PHARMACEUTIQUE SOUS FORME DE KIT COMPRENANT PROSTAGLANDIN E1

(30) Priorität: 24.03.1993 DE 4309579
(43) Veröffentlichungstag der Anmeldung: 03.01.1996
(73) Patentinhaber: SCHWARZ PHARMA AG, D-40789 Monheim/Rhld. (DE)
(72) Erfinder: SCHÜTZ, Andreas, D-50668 Köln (DE); MIKA, Hans-Jürgen, D-53229 Bonn (DE); SIEVERT, Frank, D-51399 Burscheid (DE)
(86) Internationale Anmeldenummer: DE9400325
(87) Internationale Veröffentlichungsnummer: WO9421263

(56) Entgegenhaltungen:
- EP-A- 0 150 732
- EP-A- 0 331 755
- DE-A- 4 125 255
- DATABASE WPI Week 9313, Derwent Publications Ltd., London, GB; AN 93-104185 (13) & JP,A,05 043 450 (GREEN CROSS CORP.) 23. Februar 1993

## Beschreibung

Die vorliegende Erfindung betrifft ein Kit zur Herstellung einer Prostaglandin E₁ (PGE₁) enthaltenden Fettemulsion zur direkten parenteralen Verwendung.

Insbesondere betrifft die Erfindung ein Kit, bestehend aus
a) einer wasserfreien, lyophilisierten, PGE₁ enthaltenden Emulsionszusammensetzung mindestens enthaltend ein Gefrierschulzmittel und/oder mindestens ein berüsthildner, ein polares Fett, ein Phospholipid und/oder einen hydrophilen Emulgator
b) einem wäßrigen Dispersionsmittel
   und
c) einem Filter
   zur Herstellung einer applikationsfertigen PGE₁ enthaltenden Fettemulsion, indem die wasserfreie, lyophilisierte, PGE₁ enthaltende Emulsionszusammensetzung a) mit dem wäßrigen Dispersionsmittel b) gemischt und durch den Filter c) extrudiert wird.

PGE₁ ist ein hochwirksames Gewebshormon, das erfolgreich z.B. zur Behandlung der arteriellen Verschlußkrankheit eingesetzt wird. Zur Anwendung kommt dabei ein PGE₁-α-Cyclodextrinkomplex, der in physiologischer Kochsalzlösung gelöst, parenteral, vorzugsweise intraarteriell, möglichst nahe der zu behandelnden Körperregion infundiert wird. Hohe Druckverhältnisse und geringe Verdünnungseffekte bei der intraarteriellen Infusion stellen jedoch hohe Anforderungen an die apparative Ausstattung und Qualifikation des behandelnden Arztes. Intravenöse Infusion ist zwar vergleichsweise einfacher durchzuführen, wegen lokal reizender Wirkung des PGE₁ kann jedoch auch hier nur langsam und in relativ hoher Verdünnung infundiert werden. Insgesamt führt die verlängerte Verweildauer des Wirkstoffes im Gefäßsystem vor Erreichen des Zielortes und insbesondere die zusätzliche Passage des Lungenkreislaufes zu erhöhtem Wirkstoffabbau. Sowohl intraarterielle als auch intravenöse Infusionen stellen hohe Anforderungen an Ausstattung und sorgfältige Einstellung der Infusionsrate und werden daher in der Regel stationär und nicht vom niedergelassenen Arzt durchgeführt, was einer breiten Anwendung des wertvollen Wirkstoffes in der Therapie der arteriellen Verschlußkrankheit entgegensteht.

Fettemulsionen sind disperse Systeme, in der die innere, dispergierte Phase aus sehr feinen Fettpartikeln besteht, die homogen in der aus Wasser bestehenden äußeren, geschlossenen Phase verteilt vorliegen.

Als vorwiegend lipophile Substanz kann PGE₁ teilweise oder vollständig in die innere Phase von Fettemulsionen eingebettet werden, aus der sie dann verzögert freigesetzt wird. Hierdurch werden hohe lokale Konzentrationen vermieden, der Wirkstoffabbau verringert und die Wirkdauer erhöht, so daß Einbettung in Fettemulsionen geeignet ist, die beschriebenen Probleme zu umgehen.

PGE₁ enthaltende Fettemulsionen zur parenteralen Verwendung können hergestellt werden, indem PGE₁ in der Ölphase gelöst wird, die anschließend entsprechend einem üblichen Herstellungsverfahren zu einer Fettemulsion weiterverarbeitet wird. Dies kann beispielsweise dadurch erfolgen, daß die erwärmten Öl- und Wasserphasen zunächst mit einem Mixer grob voremulgiert werden, dann mit einem Hochdruckhomogenisator feinstemulgiert werden und die erhaltene Fettemulsion anschließend mit überhitztem Wasserdampf sterilisiert wird. Feinstemulgierung ist erforderlich, um Blutdruckveränderungen und Emboliegefahr infolge großer Fetteilchen zu vermeiden. Derartige PGE₁ enthaltende Fettemulsionen sind zwar geeignet, die beschriebenen Nachteile der herkömmlichen Anwendung von PGE₁ zu lösen, weisen aber eine geringe Lagerstabilität infolge von hydrolytischem Abbau des Wirkstoffs auf, was ihrer allgemeinen Verwendbarkeit entgegensteht.

Entsprechend US-P 4,684,633 kann eine Prostaglandine enthaltende Fettemulsion, bestehend aus pflanzlichen Ölen, Phospholipiden und Wasser dadurch stabilisiert werden, indem von Phosphatidylethanolamin befreite Phospholipide verwendet werden. Wirkstoffstabilisierung wird jedoch nur für die Bedingung einer Kurzzeitsterilisation über 2,2 min bei 125°C gezeigt. Daten zur Stabilität bei Langzeitlagerung fehlen. Die Formulierung enthält auch Wasser, so daß Wirkstoffabbau infolge Hydrolyse grundsätzlich nicht ausgeschlossen werden kann.

Neben Stabilisierung des Wirkstoffes in einer applikationsfertigen Fettemulsion können Fettemulsionen mit intaktem Wirkstoff auch dadurch zur Anwendung gebracht werden, daß diese erst unmittelbar vor Gebrauch hergestellt werden. Ein Beispiel hierfür gibt die EP 0 331 755, bestehend aus Wirkstoff, Sacchariden und/oder Aminosäuren, die unmittelbar vor Anwendung vereinigt und intensiv durchgemischt werden. Intensive Durchmischung ist unbedingt erforderlich, um die Wirkstoffverteilung in die Fettemulsion zu ermöglichen. Lange Durchmischungszeiten sind bei der Anwendung jedoch von Nachteil.

Ein in EP 0 331 755, Beispiel 3, genanntes, Prostaglandine betreffendes Ausführungsbeispiel beschreibt eine Wirkstoffzusammensetzung, bestehend aus einem Prostaglandin und Triethanolamin. Triethanolamin ist jedoch physiologisch nicht unbedenklich, so daß seine Verwendung in pharmazeutischen Zubereitungen und insbesondere bei Injektabilia möglichst zu vermeiden ist.

Es bestand daher weiterhin Bedarf nach einer einfach und schnell handhabbaren Möglichkeit, eine PGE₁ enthaltende Fettemulsion zur Anwendung zu bringen. Sie sollte Hydrolyse des Wirkstoffes während der Lagerung durch Wasserfreiheit grundsätzlich ausschließen, nur physiologisch unbedenkliche Hilfsstoffe verwenden und lange Herstell- und Äquilibrierungszeiten vor der Anwendung vermeiden.

Diese Lücke im Stand der Technik konnte dadurch geschlossen werden, daß ein Kit für eine gebrauchsfähige PGE₁ enthaltende Emulsion, bestehend aus
a) einer wasserfreien, lyophilisierten, PGE₁ enthaltenden Emulsionszusammensetzung mindestens enthaltend ein Gefrierschulzmittel und/oder mindestens ein berüsthildner, ein polares Fett, ein Phospholipid und/oder einen hydrophilen Emulgator
b) einem wäßrigen Dispersionsmittel und
c) einem Filter
zur Verfügung gestellt wurde.

Dabei ist PGE₁ in einer wasserfreien Emulsionszusammensetzung enthalten, so daß Wirkstoffabbau infolge Hydrolyse grundsätzlich ausgeschlossen und die Verteilung in die Fettphase der Emulsion von Anfang an sichergestellt wird.

Vorteilhaft enthält die im Kit enthaltene PGE₁ enthaltende Emulsionszusammensetzung mindestens ein Gefrierschutzmittel und/oder mindestens einen Gerüstbildner, ein polares Fett, ein Phospholipid und/oder einen hydrophilen Emulgator. Polare Fette sind Glyceride, deren Hydroxylgruppen neben höheren Fettsäuren auch mit kurzkettigen Carbonsäuren verestert oder unverestert in freier Form vorliegen und im Gegensatz zu Triglyceriden mit höheren Fettsäuren eine gewisse Affinität zu polaren Lösungsmitteln aufweisen. Hydrophile Emulgatoren sind Tenside, deren Emulgierverhalten maßgeblich durch ihre hydrophilen Gruppen bestimmt wird und vorzugsweise Fett- in-Wasser-Emulsionen ausbilden.

Bevorzugt enthält die im Kit enthaltene PGE₁ enthaltende Emulsionszusammensetzung acetylierte Monoglyceride, Glycerinphosphatide und/oder Polyoxyethylenpolyoxypropylenpolymere.

Besonders bevorzugt enthält die im Kit enthaltene wasserfreie PGE₁ enthaltende Emulsionszusammensetzung diacetylierte Monoglyceride, Phosphatidylcholin und/oder Poloxamer 188.

Nach einer zweckmäßigen Ausführungsform liegt/liegen die Fettkomponente/n und der/die Emulgator/en in einem Gewichtsverhältnis von 1:2 bis 10:1, vorzugsweise in einem Gewichtsverhältnis von 3:2 vor.

Nach einer weiteren zweckmäßigen Ausführungsform liegt das Phospholipid und der hydrophile Emulgator in einem Gewichtsverhältnis von 3:1 bis 1:2, vorzugsweise in einem Gewichtsverhältnis von 3:2 vor.

Nach einer vorteilhaften Ausführungsform enthält die im Kit enthaltene wasserfreie, PGE₁ enthaltende Emulsionszusammensetzung als Gefrierschutzmittel/Gerüstbildner physiologisch verträgliche Mono-, Di- oder Oligosaccharide, insbesondere Trehalose und Laktose und/oder Zuckeralkohole wie Sorbit oder Mannit.

Nach einer besonders zweckmäßigen Ausführungsform enthält der Kit für eine gebrauchsfähige PGE₁ enthaltende Emulsion einen Filter, der ein Membranfilter mit einer Porengröße von 0,5-1,3 µm, vorzugsweise 0,8 µm oder ein entsprechender Tiefenfilter ist. Bevorzugt werden dabei handelsübliche Vorrichtungen, in denen der Filter fest zwischen Kanüle und Aufsteckanschluß integriert ist.

Nach einer bevorzugten Ausführungsform enthält die im Kit enthaltene wasserfreie PGE₁ enthaltende Emulsionszusammensetzung mindestens ein übliches Antioxidans, insbesondere aus der Gruppe der Tocopherole wie α-, β-, γ- oder δ-Tocopherol, vorzugsweise α-Tocopherol sowie deren physiologisch verträgliche Salze wie Phosphate, Succinate und Acetate und/oder physiologisch verträgliche Puffersalze.

Nach einer besonders bevorzugten Ausführungsform weist die innere Phase der mit dem Kit hergestellten gebrauchsfähigen Emulsion einen mittleren Teilchendurchmesser von 0,1 µm bis 5 µm, vorzugsweise 0,5 µm bis 2,0 µm auf.

Die im erfindungsgemäßen Kit enthaltene Emulsionszusammensetzung kann hergestellt werden, indem einer mittels der in der Arzneimittelherstellung gebräuchlichen Verfahren und Technologien hergestellten Emulsion die wäßrige Phase durch Lyophilisation entfernt wird.

Gegenstand der Erfindung ist daher auch ein Verfahren zur Herstellung der im erfindungsgemäßen Kit enthaltenen wasserfreien PGE₁ enthaltenden Emulsionszusammensetzung, welches dadurch gekennzeichnet ist, daß in üblicher Weise eine wirkstoffhaltige Emulsion hergestellt wird, deren äußere, wäßrige Phase anschließend mittels Gefriertrocknung entfernt wird.

Mit dem erfindungsgemäßen Kit kann in einfacher Weise eine gebrauchsfähige, d.h. zur direkten parenteralen Verabreichung geeignete PGE₁ enthaltende Emulsion hergestellt werden.

Gegenstand ist daher weiterhin ein Verfahren zur Herstellung einer gebrauchsfähigen PGE₁ enthaltenden Emulsion mittels des erfindungsgemäßen Kits, welches dadurch gekennzeichnet ist, daß die wasserfreie PGE₁ enthaltende Emulsionszusammensetzung a) mit dem wäßrigen Dispersionsmittel b) gemischt und durch den Filter c) extrudiert wird.

### Ausführungsbeispiel

2,8 g Poloxamer 188 und 40,0 g Trehalose wurden unter Erwärmen in 320 g Wasser für Injektionszwecke gelöst. Anschließend wurden 4,00 g Phosphatidylcholin, 13,0 g diacetylierte Monoglyceride, 0,20 g α-Tocopherol unter leichtem Erwärmen und Inertatmosphäre in 20,0 g absolutem Ethanol gelöst.

Die Wasserphase wurde in ein geeignetes vorsterilisiertes Reaktionsgefäß (IKA Laborreaktor LR-A 1000, Jahnke & Kunkel GmbH, Staufen, Deutschland) mit Temperiereinrichtung, Rührwerkzeug und Zahnkranzdispergierer (Ultraturrax, Jahnke & Kunkel GmbH, Staufen, Deutschland) überführt und unter Rühren bei einem Vakuum von <1 mbar auf 80°C erhitzt. Unter Aufrechterhaltung des Vakuums und Rührens wurde die ethanolische Emulgator-Lipidphase unter intensiver Homogenisation mittels Ultraturrax direkt in die Wasserphase injiziert. Anschließend wurde unter ständigem Rühren und Fortbestand des Vakuums auf Raumtemperatur abgekühlt, währenddessen in mehreren Intervallen ca. 1 Minute lang mittels Zahnkranzdispergierstab intensiv homogenisiert wurde. Der abgekühlten Emulsion wurden zur Wirkstoffeinarbeitung 131,6 mg PGE₁-α-Cyclodextrinkomplex zugegeben. Die erhaltene wirkstoffhaltige Emulsion wurde sterilfiltriert und unter aseptischen Bedingungen und Benutzung einer Dispensette mit einem Füllvolumen von 2 ml je Einzeldosis ca. 1 cm hoch in vorsterilisierte Vials überführt. Nach Befüllung wurden die Vials mit Stopfen versehen, in den Lyophilisator gestellt und über 5 Stunden bei -50°C eingefroren. Der anschließend durchgeführte Lyophilisationsprozeß erfolgte gemäß nachfolgender Tabelle:

| Zeit (Stunden) | Temperatur (°C) | Druck (µbar) |
|---|---|---|
| 24 | - 30 | 100 |
| 10 | 20 | 1 |

Anschließend wurde das Vakuum unter gleichzeitigem Einleiten von Stickstoff entfernt, die Vials durch hydraulisches Absenken der Stopfen verschlossen und dem Lyophilisator nach dessen Öffnung unter keimarmen Bedingungen entnommen.

Nach Zugabe von Wasser zerfielen die in ihnen enthaltenen Produktkuchen spontan zu einer homogenen Emulsion'. Diese wurde mittels einer Spritze über einen zwischen dieser und der Kanüle befindlichen Membranfilter mit einer Porengröße von 0,8 µm entnommen und hinsichtlich ihrer Teilchengrößen (Volumenverteilung) untersucht (Methode: Laserlichtstreuung; Malvern Master Sizer, Serie 3.01, Malvern Instruments Ltd., Spring Lane, South Malvern, Worcestershire, WR14, 1AQ, UK).

Die Messung ergab, daß 99,9 % aller Teilchen ≤ 4,10 µm sind, kein Teilchen > 5,64 µm ist und die mittlere Teilchengröße 1,15 µm beträgt. Die vorliegende Emulsion weist damit eine zur parenteralen Verabreichung geeignete Teilchengrößenverteilung auf.

## Patentansprüche

1. Kit für eine gebrauchsfähige prostaglandin E1 (PGE₁) enthaltende Emulsion, bestehend aus
a) einer wasserfreien, lyophilisierten, PGE₁ enthaltenden Emulsionszusammensetzung mindestens enthaltend ein Gefrierschutzmittel und/oder mindestens ein Gerüstbildner, ein polares Fett, ein phospholipid und/oder einen hydrophilen Emulgatar.
b) einem wäßrigen Dispersionsmittel und
c) einem Filter.

2. Kit für eine gebrauchsfähige PGE₁ enthaltende Emulsion nach Anspruch 1, dadurch gekennzeichnet, daß die wasserfreie PGE₁ enthaltende Emulsionszusammensetzung acetylierte Monoglyceride, Glycerinphosphatide und/oder Polyoxyethylenpolyoxypropylenpolymere enthält.

3. Kit für eine gebrauchsfähige PGE₁ enthaltende Emulsion nach Ansprüchen 1 bis 2, dadurch gekennzeichnet, daß die wasserfreie PGE₁ enthaltende Emulsionszusammensetzung diacetylierte Monoglyceride, Phosphatidylcholin und/oder Poloxamer 188 enthält.

4. Kit für eine gebrauchsfähige PGE₁ enthaltende Emulsion nach Ansprüchen 1 bis 3, dadurch gekennzeichnet daß Fettkomponente/n und Emulgator/en in einem Gewichtsverhältnis von 1: 2 bis 10 : 1, vorzugsweise in einem Gewichtsverhältnis von 3:2, vorliegen.

5. Kit für eine gebrauchsfähige PGE₁ enthaltende Emulsion nach Ansprüchen 1 bis 4, dadurch gekennzeichnet, daß das Phospholipid und der hydrophile Emulgator in einem Gewichtsverhältnis von 3:1 bis 1:2, vorzugsweise in einem Gewichtsverhältnis von 3:2, vorliegen.

6. Kit für eine gebrauchsfähige PGE₁ enthaltende Emulsion nach Ansprüchen 1 bis 5, dadurch gekennzeichnet, daß die wasserfreie PGE₁ enthaltende Emulsionszusammensetzung als Gefrierschutzmittel/Gerüstbildner physiologisch verträgliche Mono-, Di- oder Oligosaccharide, insbesondere Trehalose und Laktose und/oder Zuckeralkahole wie Sorbit und Mannit enthält.

7. Kit für eine gebrauchsfähige PGE₁ enthaltende Emulsion nach Ansprüchen 1 bis 6, dadurch gekennzeichnet, daß der Filter ein Membranfilter mit einer Porengröße von 0,5-1,3 µm, vorzugsweise 0,8 µm oder ein entsprechender Tiefenfilter ist und vorzugsweise fest zwischen Kanüle und Aufsteckadapter integriert ist.

8. Kit für eine gebrauchsfähige PGE₁ enthaltende Emulsion nach Ansprüchen 1 bis 7, dadurch gekennzeichnet, daß die wasserfreie PGE₁ enthaltende Emulsionszusammensetzung mindestens ein übliches Antioxidans, insbesondere aus der Gruppe der Tocopherole wie α, β, γ oder δ-Tocopherol, vorzugsweise α-Tocopherol sowie deren physiologisch verträgliche Salze wie Phosphate, Succinate und Acetate und/oder physiologisch verträgliche Puffersalze enthält.

9. Kit für eine gebrauchsfähige PGE₁ enthaltende Emulsion nach Ansprüchen 1 bis 8, dadurch gekennzeichnet, daß die innere Phase der Emulsion, einen mittleren Teilchendurchmesser von 0,1 µm bis 5 µm, vorzugsweise 0,5 µm bis 2,0 µm, aufweist.

10. Verfahren zur Herstellung einer wasserfreien Emulsionszusammensetzung als Teil eines Kits für eine gebrauchsfähige PGE₁ enthaltende Emulsion nach einem der Ansprüche 1 bis 9, dadurch gekennzeichnet, daß in üblicher Weise eine wirkstoffhaltige Emulsion hergestellt wird, deren äußere, wäßrige Phase anschließend mittels Gefriertrocknung entfernt wird.

11. Verfahren zur Herstellung einer gebrauchsfähigen PGE₁ enthaltenden Emulsion mittels eines Kits für eine gebrauchsfähige PGE₁ enthaltende Emulsion nach einem der Ansprüche 1 bis 10 dadurch gekennzeichnet, daß die wasserfreie PGE₁ enthaltende Emulsionszusammensetzung a) mit dem wäßrigen Dispersionsmittel b) gemischt und durch den Filter c) extrudiert wird.

## Claims

1. A kit for a usable emulsion which contains prostaglandin E₁ (PGE₁), consisting of
a) an anhydrous, lyophilised emulsion composition which contains PGE₁ and which contains at least one antifreeze agent and/or at least one framework former, a polar fat, a phospholipid and/or a hydrophilic emulsifier,
b) an aqueous dispersing agent, and
c) a filter.

2. A kit for a usable emulsion which contains PGE₁ according to claim 1, characterised in that the anhydrous emulsion composition which contains PGE₁ contains acetylated monoglycerides, glycerol phosphatides and/or polyoxyethylene-polyoxypropylene polymers.

3. A kit for a usable emulsion which contains PGE₁ according to claims 1 and 2, characterised in that the anhydrous emulsion composition which contains PGE₁ contains diacetylated monoglycerides, phosphatidylcholine and/or Poloxamer 188.

4. A kit for a usable emulsion which contains PGE₁ according to claims 1 to 3, characterised in that fat component(s) and emulsifier(s) are present in a ratio by weight of 1:2 to 10:1, preferably in a ratio by weight of 3:2.

5. A kit for a usable emulsion which contains PGE₁ according to claims 1 to 4, characterised in that the phospholipid and the hydrophilic emulsifier are present in a ratio by weight of 3:1 to 1:2, preferably in a ratio by weight of 3:2.

6. A kit for a usable emulsion which contains PGE₁ according to claims 1 to 5, characterised in that the anhydrous emulsion composition which contains PGE₁ contains physiologically compatible mono-, di- or oligosaccharides, particularly trehalose and lactose and/or sugar alcohols such as sorbitol and mannitol, as antifreeze agents/framework formers.

7. A kit for a usable emulsion which contains PGE₁ according to claims 1 to 6, characterised in that the filter is a membrane filter with a pore size of 0.5 - 1.3 µm, preferably of 0.8 µm, or is a corresponding deep-bed filter, and is preferably fixedly incorporated between a cannula and a push-on adaptor.

8. A kit for a usable emulsion which contains PGE₁ according to claims 1 to 7, characterised in that the anhydrous emulsion composition which contains PGE₁ contains at least one customary antioxidant, particularly from the tocopherol group, such as α-, β-, γ- or δ-tocopherol, preferably α-tocopherol, as well as physiologically compatible salts thereof such as phosphates, succinates and acetates, and/or physiologically compatible buffer salts.

9. A kit for a usable emulsion which contains PGE₁ according to claims 1 to 8, characterised in that the internal phase of the emulsion has an average particle diameter of 0.1 µm to 5 µm, preferably of 0.5 µm to 2.0 µm.

10. A method of producing an,anhydrous emulsion composition as part of a kit for a usable emulsion which contains PGE₁ according to any one of claims 1 to 9, characterised in that an emulsion which contains an active ingredient is produced in a customary manner, the external aqueous phase of which emulsion is subsequently removed by freeze-drying.

11. A method of producing a usable emulsion composition which contains PGE₁ by means of a kit for a usable emulsion which contains PGE₁ according to any one of claims 1 to 10, characterised in that the anhydrous emulsion composition a) which contains PGE₁ is mixed with the aqueous dispersing agent b) and is extruded through the filter c).

## Revendications

1. Kit pour une émulsion prête à l'emploi contenant de la prostaglandine E₁ (PGE₁), composé de:
a) une composition d'émulsion contenant de la PGE₁ anhydre, lyophilisée, contenant au moins un agent de cryoprotection et/ou au moins un agent d'ossature, une graisse polaire, un phospholipide et/ou un émulsifiant hydrophile,
b) un dispersant aqueux, et
c) un filtre.

2. Kit pour une émulsion prête à l'emploi contenant de la PGE₁ selon la Revendication 1, ***caractérisé en ce que*** la composition d'émulsion contenant de la PGE₁ contenue dans le kit comprend des monoglycérides acétylés, des glycérol-phosphatides et/ou des polymères polyoxyéthylène - polyoxypropylène.

3. Kit pour une émulsion prête à l'emploi contenant de la PGE₁ selon les Revendications 1 à 2, ***caractérisé en ce que*** la composition d'émulsion contenant de la PGE₁ contenue dans le kit comprend des monoglycérides diacétylés, de la phosphatidylcholine et/ou du Poloxamer 188.

4. Kit pour une émulsion prête à l'emploi contenant de la PGE₁ selon les Revendications 1 à 3, ***caractérisé en ce que*** la/les composant(s) gras(s) et le(s) émulsifiant(s) sont présents suivant un rapport pondéral de 1/2 à 10/1, de préférence selon un rapport pondéral de 3/2.

5. Kit pour une émulsion prête à l'emploi contenant de la PGE₁ selon les Revendications 1 à 4, ***caractérisé en ce que*** le phospholipide et l'émulsifiant hydrophile sont présents suivant un rapport pondéral de 3/1 à 1/2, de préférence selon un rapport pondéral de 3/2.

6. Kit pour une émulsion prête à l'emploi contenant de la PGE₁ selon les Revendications 1 à 5, ***caractérisé en ce que*** la composition d'émulsion anhydre contenant de la PGE₁ contient comme agent de cryoprotection des mono-, di- ou oligosaccharides physiologiquement compatibles, en particulier tréhalose et lactose et/ou des alcools de sucre comme sorbitol ou mannitol.

7. Kit pour une émulsion prête à l'emploi contenant de la PGE₁ selon les Revendications 1 à 6, ***caractérisé en ce que*** le filtre est un filtre à membrane d'une taille de pores de 0,5 à 1,3 µm, de préférence 0,8 µm, ou un filtre à lit profond correspondant et est intégré de préférence de manière fixe entre la canule et l'embout.

8. Kit pour une émulsion prête à l'emploi contenant de la PGE₁ selon les Revendications 1 à 7, ***caractérisé en ce que*** la composition d'émulsion anhydre contenant de la PGE₁ contient au moins un antioxydant usuel, en particulier du groupe des tocophérols tels que α-, β-, γ- ou δ-to-cophérol, de préférence α-tocophérol, ainsi que leurs sels physiologiquement compatibles, tels que des phosphates, des succinates et des acétates et/ou des sels tampons physiologiquement compatibles.

9. Kit pour une émulsion prête à l'emploi contenant de la PGE₁ selon les Revendications 1 à 8, ***caractérisé en ce que*** la phase interne de l'émulsion présente un diamètre moyen de particules de 0,1 µm à 5 µm, de préférence 0,5 µm à 2,0 µm.

10. Procédé de préparation d'une composition d'émulsion anhydre faisant partie d'un kit pour une émulsion prête à l'emploi contenant de la PGE₁ selon l'une quelconque des Revendications 1 à 9, ***caractérisé en ce qu'on*** prépare de manière habituelle une émulsion contenant un principe actif, dont on élimine ensuite la phase extérieure aqueuse par lyophilisation.

11. Procédé de préparation d'une émulsion anhydre au moyen d'un kit pour une émulsion prête à l'emploi contenant de la PGE₁ selon l'une quelconque des Revendications 1 à 10, ***caractérisé en ce que*** la composition d'émulsion anhydre contenant de la PGE₁ : (a) est mélangée à l'agent de dispersion aqueux (b) et extrudée à travers le filtre (c).
